(19) Europäisches Patentamt
European Patent Office
Office européen des brevets

(11) EP 2 406 729 B1

(12) **EUROPEAN PATENT SPECIFICATION**

(45) Date of publication and mention of the grant of the patent:
**05.07.2017 Bulletin 2017/27**

(21) Application number: **10751102.4**

(22) Date of filing: **10.03.2010**

(51) Int Cl.:
***G06F 19/24*** *(2011.01)* ***G06F 19/20*** *(2011.01)*
***C12Q 1/68*** *(2006.01)* *G06F 19/18* *(2011.01)*

(86) International application number:
**PCT/SG2010/000079**

(87) International publication number:
**WO 2010/104473 (16.09.2010 Gazette 2010/37)**

(54) **A METHOD, SYSTEM AND COMPUTER PROGRAM PRODUCT FOR THE SYSTEMATIC EVALUATION OF THE PROGNOSTIC PROPERTIES OF GENE PAIRS FOR MEDICAL CONDITIONS.**

VERFAHREN, SYSTEM UND COMPUTERPROGRAMMPRODUKT FÜR DIE SYSTEMATISCHE BEURTEILUNG DER PROGNOSTISCHEN EIGENSCHAFTEN VON GENPAAREN FÜR KRANKHEITSZUSTÄNDE.

PROCÉDÉ, SYSTÈME ET PRODUIT PROGRAMME D'ORDINATEUR D'ÉVALUATION SYSTÉMATIQUE DES PROPRIÉTÉS PRONOSTIQUES DE PAIRES DE GÈNES POUR DES MALADIES.

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **10.03.2009 US 158948 P**

(43) Date of publication of application:
**18.01.2012 Bulletin 2012/03**

(73) Proprietor: **Agency for Science, Technology and Research**
**Singapore 138632 (SG)**

(72) Inventors:
- **MILLER, Lance, D.**
  **Singapore 138672 (SG)**
- **KARUTURI, Krishna, Murthy**
  **Singapore 138672 (SG)**

(74) Representative: **Bailey, Jennifer Ann et al**
**Marks & Clerk LLP**
**Alpha Tower**
**Suffolk Street Queensway**
**Birmingham B1 1TT (GB)**

(56) References cited:

- **MOTAKIS E ET AL: "Identification of essential genes and gene pairs associated with survival time of cancer patients", PROCEEDINGS OF THE 2007 INTERNATIONAL CONFERENCE ON BIOINFORMATICS & COMPUTATIONAL BIOLOGY, BIOCOMP 2007 : [AT] WORLDCOMP'07, JUNE 25 - 28, 2007, LAS VEGAS, NEVADA, USA, CSREA PRESS, vol. 2, 1 January 2007 (2007-01-01), pages 753-759, XP009169912, ISBN: 1-60132-040-X**
- **KUZNETSOV V A ET AL: "Low and high-agressive genetic breast cancer subtypes and significant survival gene signatures", NEURAL NETWORKS, 2008. IJCNN 2008. (IEEE WORLD CONGRESS ON COMPUTATIONAL INTELLIGENCE). IEEE INTERNATIONAL JOINT CONFERENCE ON, IEEE, PISCATAWAY, NJ, USA, 1 June 2008 (2008-06-01), pages 4151-4156, XP031328132, ISBN: 978-1-4244-1820-6**
- **ROSELL RAFAEL ET AL: "BRCA1: a novel prognostic factor in resected non-small-cell lung cancer", PLOS ONE, PUBLIC LIBRARY OF SCIENCE, US, vol. 2, no. 11, 7 November 2007 (2007-11-07), pages e1129-1, XP002490290, ISSN: 1932-6203, DOI: 10.1371/JOURNAL.PONE.0001129**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 406 729 B1

- P. BROET ET AL: "Identifying gene expression changes in breast cancer that distinguish early and late relapse among uncured patients", BIOINFORMATICS, vol. 22, no. 12, 15 June 2006 (2006-06-15) , pages 1477-1485, XP55064274, ISSN: 1367-4803, DOI: 10.1093/bioinformatics/btl110
- GOETZ ET AL.: 'A Two-Gene Expression Ratio of Homeobox 13 and Interleukin-17B Receptor for Prediction of Recurrence and Survival in Women Receiving Adjuvant Tamoxifen' CLINICAL CANCER RESEARCH, [Online] vol. 12, no. 7, 01 April 2006, pages 2080 - 2087, XP002572497 Retrieved from the Internet: <URL:http://clincancerres.aacrjournals.org/content/12/7/2080.full.pdf> [retrieved on 2010-05-26]
- ROSS ET AL.: 'Commercialized Multigene Predictors of Clinical Outcome for Breast Cancer' THE ONCOLOGIST, [Online] vol. 13, no. 5, May 2008, pages 477 - 493, XP002541429 Retrieved from the Internet: <URL:http://theoncologist.alphamedpress.org/ogi/reprint/13/5/477.pdf> [retrieved on 2010-04-15]

SEQUENCE LISTING

<110> Agency for Science, Technology and Research

<120> A method for the systematic evaluation of the prognostic properties of gene pairs for medical conditions, and certain gene pairs identified

<130> FP4650

<150> US 61/158,948
<151> 2009-03-10

<160> 6

<170> Patent In version 3.4

<210> 1
<211> 2953
<212> DNA
<213> Homo sapiens

<400> 1

```
atatagagac gtttccgcct cctgcttgaa actaacccct ctttttctcc aaaggagtgc     60
ttgtggagat cggatctttt ctccagcaat tgggggaaag aaggcttttt ctctgaattc    120
gcttagtgta accagcggcg tatatttttt aggcgccttt tcgaaaacct agtagttaat    180
attcatttgt ttaaatctta ttttattttt aagctcaaac tgcttaagaa taccttaatt    240
ccttaaagtg aaataatttt ttgcaaaggg gtttcctcga tttggagctt ttttttttctt    300
ccaccgtcat ttctaactct taaaaccaac tcagttccat catggtgatg ttcaagaaga    360
tcaagtcttt tgaggtggtc tttaacgacc ctgaaaaggt gtacggcagt ggcgagaagg    420
tggctggccg ggtgatagtg gaggtgtgtg aagttactcg tgtcaaagcc gttaggatcc    480
tggcttgcgg agtggctaaa gtgctttgga tgcagggatc ccagcagtgc aaacagactt    540
cggagtacct gcgctatgaa gacacgcttc ttctggaaga ccagccaaca ggtgagaatg    600
agatggtgat catgagacct ggaaacaaat atgagtacaa gttcggcttt gagcttcctc    660
aggggcctct gggaacatcc ttcaaaggaa aatatgggtg tgtagactac tgggtgaagg    720
cttttcttga ccgcccgagc cagccaactc aagagacaaa gaaaaacttt gaagtagtgg    780
atctggtgga tgtcaatacc cctgatttaa tggcacctgt gtctgctaaa aaagaaaaga    840
aagtttcctg catgttcatt cctgatgggc gggtgtctgt ctctgctcga attgacagaa    900
aaggattctg tgaaggtgat gagatttcca tccatgctga ctttgagaat acatgttccc    960
gaattgtggt ccccaaagct gccattgtgg cccgccacac ttaccttgcc aatggccaga   1020
ccaaggtgct gactcagaag ttgtcatcag tcagaggcaa tcatattatc tcaggacat    1080
```

gcgcatcatg gcgtggcaag agccttcggg ttcagaagat caggccttct atcctgggct 1140
gcaacatcct tcgagttgaa tattccttac tgatctatgt tagcgttcct ggatccaaga 1200
aggtcatcct tgacctgccc ctggtaattg gcagcagatc aggtctaagc agcagaacat 1260
ccagcatggc cagccgaacc agctctgaga tgagttgggt agatctgaac atccctgata 1320
ccccagaagc tcctccctgc tatatggatg tcattcctga agatcaccga ttggagagcc 1380
caaccactcc tctgctagat gacatggatg gctctcaaga cagccctatc tttatgtatg 1440
cccctgagtt caagttcatg ccaccaccga cttatactga ggtggatccc tgcatcctca 1500
acaacaatgt gcagtgagca tgtggaagaa aagaagcagc tttacctact tgtttctttt 1560
tgtctctctt cctggacact cactttttca gagactcaac agtctctgca atggagtgtg 1620
ggtccacctt agcctctgac ttcctaatgt aggaggtggt cagcaggcaa tctcctgggc 1680
cttaaaggat gcggactcat cctcagccag cgcccatgtt gtgatacagg ggtgtttgtt 1740
ggatgggttt aaaaataact agaaaaactc aggcccatcc attttctcag atctccttga 1800
aaattgaggc cttttcgata gtttcgggtc aggtaaaaat ggcctcctgg cgtaagcttt 1860
tcaaggtttt ttggaggctt tttgtaaatt gtgataggaa ctttggacct tgaacttatg 1920
tatcatgtgg agaagagcca atttaacaaa ctaggaagat gaaaagggaa attgtggcca 1980
aaactttggg aaaaggaggt tcttaaaatc agtgtttccc ctttgtgcac ttgtagaaaa 2040
aaaagaaaaa ccttctagag ctgatttgat ggacaatgga gagagctttc cctgtgatta 2100
taaaaaagga agctagctgc tctacggtca tctttgctta agagtatact ttaacctggc 2160
ttttaaagca gtagtaactg ccccaccaaa ggtcttaaaa gccatttttg gagcctattg 2220
cactgtgttc tcctactgca aatattttca tatgggagga tggttttctc ttcatgtaag 2280
tccttggaat tgattctaag gtgatgttct tagcacttta attcctgtca aatttttttgt 2340
tctccccttc tgccatctta aatgtaagct gaaactggtc tactgtgtct ctagggttaa 2400
gccaaaagac aaaaaaaatt ttactacttt tgagattgcc ccaatgtaca gaattatata 2460
attctaacgc ttaaatcatg tgaaagggtt gctgctgtca gccttgccca ctgtgacttc 2520
aaacccaagg aggaactctt gatcaagatg ccgaaccctg tgttcagaac ctccaaatac 2580
tgccatgaga aactagaggg caggtcttca taaaagccct ttgaaccccc ttcctgccct 2640
gtgttaggag atagggatat tggcccctca ctgcagctgc cagcacttgg tcagtcactc 2700
tcagccatag cactttgttc actgtcctgt gtcagagcac tgagctccac ccttttctga 2760
gagttattac agccagaaag tgtgggctga agatggttgg tttcatgttt ttgtattatg 2820
tatctttttg tatggtaaag actatatttt gtacttaacc agatatattt ttaccccaga 2880

tggggatatt ctttgtaaaa aatgaaaata aagttttttt aatggaaaaa aaaaaaaaaa 2940

aaaaaaaaaa aaa 2953

<210> 2
<211> 542
<212> DNA
<213> Homo sapiens

<220>
<221> misc_feature
<222> (523)..(523)
<223> n is a, c, g, or t

<220>
<221> misc_feature
<222> (535)..(535)
<223> n is a, c, g, or t

<400> 2

atataatgag agatataatt tttgttaata agacaaaggt aatatattgg atacaaagac 60

acaaatgtat tgtgtgttca attattttgt tgtcttgaga tttaatattc tttccaagag 120

cttttaatga agcagagagc tagtacttca ttttcactgg atacattttc agcatcatga 180

gttgtcacag cctctgagcc cctgatctga agccagaagg gctgagtgta ttgtaaactt 240

attcttgcat gttgctgtct gggaatggac cacactacag caggtagttc tgggggcgat 300

actgccgaaa ggcccgaaca catgtatttt ggctgcaatt gaggaacttg ggatgctatt 360

aattttgtat ttcagcaact gccccttctc ctatcccaaa gcaccaatta ctgccctctg 420

cctcagcagt accagtataa gatgacattc caaagactgg aggcaactca gcctgagtta 480

attcacaaaa ttatgccatg ctggggcttg agcttgagct tgngcttagg cttgngctca 540

gc 542

<210> 3
<211> 2130
<212> DNA
<213> Homo sapiens

<400> 3

ggttaaacgg ggcccaaggc aggggtggcg ggtcagtgct gctcgggggc ttctccatcc 60

aggtccctgg agttcctggt ccctggagct ccgcacttgg cggcgcaacc tgcgtgaggc 120

agcgcgactc tggcgactgg ccggccatgc cttcccgggc tgaggactat gaagtgttgt 180

acaccattgg cacaggctcc tacggccgct gccagaagat ccggaggaag agtgatggca 240

agatattagt ttggaaagaa cttgactatg gctccatgac agaagctgag aaacagatgc 300

ttgtttctga agtgaatttg cttcgtgaac tgaaacatcc aaacatcgtt cgttactatg 360
atcggattat tgaccggacc aatacaacac tgtacattgt aatggaatat tgtgaaggag 420
gggatctggc tagtgtaatt acaaagggaa ccaaggaaag gcaatactta gatgaagagt 480
ttgttcttcg agtgatgact cagttgactc tggccctgaa ggaatgccac agacgaagtg 540
atggtggtca taccgtattg catcgggatc tgaaaccagc caatgttttc ctggatggca 600
agcaaaacgt caagcttgga gactttgggc tagctagaat attaaaccat gacacgagtt 660
ttgcaaaaac atttgttggc acaccttatt acatgtctcc tgaacaaatg aatcgcatgt 720
cctacaatga gaaatcagat atctggtcat tgggctgctt gctgtatgag ttatgtgcat 780
taatgcctcc atttacagct tttagccaga aagaactcgc tgggaaaatc agagaaggca 840
aattcaggcg aattccatac cgttactctg atgaattgaa tgaaattatt acgaggatgt 900
taaacttaaa ggattaccat cgaccttctg ttgaagaaat tcttgagaac cctttaatag 960
cagatttggt tgcagacgag caaagaagaa atcttgagag aagagggcga caattaggag 1020
agccagaaaa atcgcaggat tccagccctg tattgagtga gctgaaactg aaggaaattc 1080
agttacagga gcgagagcga gctctcaaag caagagaaga aagattggag cagaaagaac 1140
aggagctttg tgttcgtgag agactagcag aggacaaact ggctagagca gaaaatctgt 1200
tgaagaacta cagcttgcta aaggaacgga agttcctgtc tctggcaagt aatccagaac 1260
ttcttaatct tccatcctca gtaattaaga agaaagttca tttcagtggg gaaagtaaag 1320
agaacatcat gaggagtgag aattctgaga gtcagctcac atctaagtcc aagtgcaagg 1380
acctgaagaa aaggcttcac gctgcccagc tgcgggctca agccctgtca gatattgaga 1440
aaaattacca actgaaaagc agacagatcc tgggcatgcg ctagccaggt agagagacac 1500
agagctgtgt acaggatgta atattaccaa cctttaaaga ctgatattca aatgctgtag 1560
tgttgaatac ttggttccat gagccatgcc tttctgtata gtacacatga tatttcggaa 1620
ttggttttac tgttcttcag caactattgt acaaaatgtt cacatttaat ttttctttct 1680
tcttttaaga acatattata aaaagaatac tttcttggtt gggcttttaa tcctgtgtgt 1740
gattactagt aggaacatga gatgtgacat tctaaatctt gggagaaaaa ataatgttag 1800
gaaaaaaata tttatgcagg aagagtagca ctcactgaat agttttaaat gactgagtgg 1860
tatgcttaca attgtcatgt ctagatttaa attttaagtc tgagatttta aatgtttttg 1920
agcttagaaa acccagttag atgcaatttg gtcattaata ccatgacatc ttgcttataa 1980
atattccatt gctctgtagt tcaaatctgt tagctttgtg aaaattcatc actgtgatgt 2040

```
ttgtattctt tttttttttc tgtttaacag aatatgagct gtctgtcatt tacctacttc   2100

tttcccacta aataaaagaa ttcttcagtt                                     2130
```

<210> 4
<211> 570
<212> DNA
<213> Homo sapiens

<220>
<221> misc_feature
<222> (3)..(4)
<223> n is a, c, g, or t

<220>
<221> misc_feature
<222> (7)..(7)
<223> n is a, c, g, or t

<220>
<221> misc_feature
<222> (68)..(68)
<223> n is a, c, g, or t

<220>
<221> misc_feature
<222> (237)..(237)
<223> n is a, c, g, or t

<220>
<221> misc_feature
<222> (243)..(243)
<223> n is a, c, g, or t

<220>
<221> misc_feature
<222> (252)..(252)
<223> n is a, c, g, or t

<220>
<221> misc_feature
<222> (427)..(427)
<223> n is a, c, g, or t

<220>
<221> misc_feature
<222> (500)..(500)
<223> n is a, c, g, or t

<400> 4

```
ttnngtncga caccagcaca ccgcggacac ctagctagca gaccggtgtg gcttgctctt      60
taggggtnat gcaaaagaat tatcccccag aaaaaaaatc cccaaattat cccagacagg     120
cagctccgtg gaggtgacaa ggtgagaggg agaggagggg aggaggcccc aggacgccag     180
ggtggccggc tgctgggcca cgtgctgtcc gtggtgagcc ctgccgctgt cccatgnccc     240
agngagccac tngtgcggag ccggcagatg tttaccctgt gttcatggat ggggacagct     300
gtcctgggca cagcgtggac ggaagatggt gtccactctg agtgttcatc ggtggacagt     360
cccctttgga gtttaggatc ctgtgtcctt gttcccattc tgtgcccctc cctttggctt     420
gaatttntag gggaagataa aggaaatcct tgtttgaaaa aaatgtgaat ttaaaagtgg     480
gcaaggcaat tgggcaaggn agcccttaac ccttttgagt aaggggtttt aaaccacccc     540
accaaacctg ggttaagcca cccttgaagc                                      570
```

<210> 5
<211> 1601
<212> DNA
<213> Homo sapiens

<400> 5

ggagttacgt agaagacagg gcgtcctggt gtacgggctg gccctggacg tcacttccgc 60
tggcggcagt ggcgtcactt cctgctcttg gggcggggc atccgtgtcc ttgcggtgct 120
gggcagcaga ccgtccaaac cgacacgcgt ggtatcctcg cggtgtccgg caagagacta 180
ccaagacaga cgctatgact gaggctgatg tgaatccaaa ggcctatccc cttgccgatg 240
cccacctcac caagaagcta ctggacctcg ttcagcagtc atgtaactat aagcagcttc 300
ggaaaggagc caatgaggcc accaaaaccc tcaacagggg catctctgag ttcatcgtga 360
tggctgcaga cgccgagcca ctggagatca ttctgcacct gccgctgctg tgtgaagaca 420
agaatgtgcc ctacgtgttt gtgcgctcca agcaggccct ggggagagcc tgtggggtct 480
ccaggcctgt catcgcctgt tctgtcacca tcaaagaagg ctcgcagctg aaacagcaga 540
tccaatccat tcagcagtcc attgaaaggc tcttagtcta aacctgtggc ctctgccacg 600
tgctccctgc cagcttcccc cctgaggttg tgtatcatat tatctgtgtt agcatgtagt 660
atttcagct actctctatt gttataaaat gtagtactaa atctggtttc tggatttttg 720
tgttgttttt gttctgtttt acagggttgc tatcccctt cctttcctcc ctccctctgc 780
catccttcat ccttttatcc tcccttttg gaacaagtgt tcagagcaga cagaagcagg 840
gtggtggcac cgttgaaagg cagaaagagc caggagaaag ctgatggagc caggacagag 900
atctggttcc agctttcagc cactagcttc ctgttgtgtg cggggtgtgg tggaattaaa 960
cagcattcat tgtgtgtccc tgtgcctggc acacagaatc attcatacgt gttcaagtga 1020
tcaaggggtt tcatttgctc ttgggggatt aggtatcatt tggggaggaa gcatgtgttc 1080
tgtgaggttg ttcggctatg tccaagtgtc gtttactaat gtacccctgc tgtttgcttt 1140
tggtaatgtg atgttgatgt tctcccccta cccacaacca tgcccttgag ggtagcaggg 1200

cagcagcata ccaaagagat gtgctgcagg actccggagg cagcctgggt gggtgagcca 1260
tggggcagtt gacctgggtc ttgaaagagt cgggagtgac aagctcagag agcatgaact 1320
gatgctggca tgaaggattc caggaagatc atggagacct ggctggtagc tgtaacagag 1380
atggtggagt ccaaggaaac agcctgtctc tggtgaatgg gactttcttt ggtggacact 1440
tggcaccagc tctgagagcc cttcccctgt gtcctgccac catgtgggtc agatgtactc 1500
tctgtcacat gaggagagtg ctagttcatg tgttctccat tcttgtgagc atcctaataa 1560
atctgttcca ttttgatgac aaaaaaaaaa aaaaaaaaaa a 1601

<210> 6
<211> 1180
<212> DNA
<213> Homo sapiens

<400> 6

```
ccgggtcact gttaaggggg ccgccatggc ccggctgtgg ggcgcgctga gtctttggcc      60
actgtgggcg gccgtgccgt ggggcggggc ggcagccgtc ggtgtccggg cttgcagctc     120
cacggccgcc ccggacggcg tcgagggccc ggcgctgcgg cgctcctatt ggcgccacct     180
gaggcgtctg gtgctgggtc ctcccgaacc gccgttctcg cacgtgtgcc aagtcgggga     240
cccggtgctg cgcggcgtgg cggccccggt ggagcgggcg cagctaggcg ggcccgagct     300
gcagcggctg acgcaacggc tggtccaggt gatgcggcgg cggcgctgcg tgggcctaag     360
cgcgccgcag ctgggggtgc cgcggcaggt gctggcgctg gagctccccg aggcgctgtg     420
tcgggagtgc ccgccccgcc agcgcgcgct ccgccaaatg gagcccttcc ccctgcgcgt     480
gttcgtgaac cccagcctgc gagtgcttga cagccgcctg gtcacctttc ccgagggctg     540
cgagagcgtc gccggcttcc tggcctgcgt gccccgcttc caggcggtgc agatctcagg     600
gctggacccc aatggagaac aggtggtgtg gcaggcgagc gggtgggcag cccgcatcat     660
ccagcacgag atggaccacc tgcagggctg cctgtttatt gacaaaatgg acagcaggac     720
gttcacaaac gtctattgga tgaaggtgaa tgactaaagc tttgctactg gggctgagga     780
ttccggatac caagacgcaa acactttcac tttgagctgg gcaaatctta cttggcatca     840
acttggatgg ctcgcatatg acaggaaact ggattgccaa ggcatggcag actgagctgg     900
aggaagatgt cagaaatgtt tgccctgaaa tcagttacgg acaaaatgtt ggctacaact     960
tgaggagaaa aatcaccccc aaaggagtgg acatttccta acaattctgt atggaggaag    1020
gtggggtaat tgcattcgtc tgcagtagac acgagttcct cggacctgta taatctccca    1080
aagccaaggt ttggtaataa tgtagtcccc aaatacctga aagctgtctt taaaaatgca    1140
ggtaagcatg tgactggcaa aaaaaaaaaa aaaaaaaaaa                          1180
```

## Description

### Field of the invention

The present invention relates to identification of pairs of genes for which the respective gene expression values in a subject are statistically significant in relation to a medical condition, for example cancer, or more particularly breast cancer. The gene expression values may for example be indicative of the susceptibility of the subject to the medical condition, or the prognosis of a subject who exhibits the medical condition. The invention further relates to methods and arrays employing the identified gene pairs, and in particular three specific gene pairs obtained by the method, in obtaining information about specific subjects.

### Background of the invention

Global gene expression profiles of subjects are often used to obtain information about those subjects, such as their susceptibility to certain medical condition, or, in the case of subjects exhibiting medical conditions, their prognosis. For example, having determined that a particular gene is important, the level in which that gene is expressed in a subject can be used to classify the individual into one of a plurality of classes, each class being associated with a different susceptibility or prognosis. An important task is the identification of "significant gene signature(s)", that is gene set(s) such that the corresponding gene expression values can be such to classify subjects in a useful way.

Motakis et al. ("Identification of essential genes and gene pairs associated with survival time of cancer patients", BIOCOMP, page 753-759. CSREA Press, (2007*))* describes a method of identifying genes and gene pairs associated with survival time of cancer patients which applies absolute gene expression cut-offs $c^1$ and $c^2$ for each gene pair $i^1$ and $i^2$ and fits a Cox proportional hazards model for each combination of possibilities.

V. Kuznetsov et al. ("Low and high-aggressive genetic breast cancer subtypes and significant survival gene signatures", IJCNN 2008, pages 4151-4156) describes a method of identifying "survival significant" genes, by fitting, for each gene *i*, a Cox proportional hazards model to clinical event data based on an absolute expression level cut-off $c^i$.

Rosell et al. ("BRCA1: a novel prognostic factor in resected non-small-cell lung cancer", PLOS ONE, vol. 2, no. 11, pages e1129-1) discloses forward and backward Cox regression analysis based on expression levels of previously selected genes.

Broët et al. ("Identifying gene expression changes in breast cancer that distinguish early and late relapse among uncured patients", Bioinformatics, vol. 22, no. 12, pages 1477-1485) discloses a method of distinguishing, on the basis of gene expression, between early and late relapse among uncured patients.

Goetz et al. ("A Two-Gene Expression Ratio of Homeobox 13 and Interleukin-17B Receptor for Prediction of Recurrence and Survival in Women Receiving Adjuvant Tamoxifen", Clinical Cancer Research, vol. 12, no. 7, 2006, pages 2080-2087) discloses a pair of genes, *HOXB13* and *IL-17BR,* for which the expression ratio can be used as a predictor of recurrence and survival in women receiving adjuvant tamoxifen.

### 1. The theory of survival analysis

First we will describe briefly the background theory of survival analysis. We denote by T the patient's *survival time. T* is a continuous non-negative random variable which can take values *t,* $t \in [0,\infty)$. T has density function *f(t)* and cumulative distribution function $F(t)=P(T \le t)$. $F(t)=\int_0^t f(t')dt'$. We are primarily interested in estimating two quantities:

The survival function:

$$S(t)=P(T>t)=1-F(t)$$

The hazard function:

$$h(t)=\frac{f(t)}{S(t)}=\lim_{\Delta t\to 0}\frac{P(t\le T<(t+\Delta t)|T\ge t)}{\Delta t}$$

The survival function expresses the probability of a patient to be alive at time t. It is often presented in the form

$S(t) = exp(-H(t))$, where $H(t)=\int_0^t h(u)du$ denotes the cumulative hazard. The hazard function assesses the instantaneous risk of death at time t, conditional on survival up to that time.

Notice that the hazard function is expressed in terms of the survival function. To this extent, survival distributions and hazard functions can be generated for any distribution defined for $t \in [0,\infty)$. By considering a random variable W, distributed in $(\infty,-\infty)$, we can generate a family of survival distributions by introducing location ($\alpha$) and scale ($\sigma$) changes of the form $\log T = \alpha + \sigma W$.

Alternatively, we can express the relationship of the survival distribution to covariates by means of a parametric model. The parametric model employs a "regressor" variable *x*. Take for example a model based on the exponential distribution and write: $\log(h(t)) = \alpha + \beta x$, or equivalently, $h(t) = \exp(\alpha + \beta x)$.
This is a linear model for the log-hazard, or, equivalently, a multiplicative model for the hazard. The constant $\alpha$ represents the log-baseline hazard (the hazard when the regressor $x = 0$) and the slope parameter $\beta$ gives the change in hazard rate as *x* varies. This is an easy example of how survival models can be obtained from simple distributional assumptions. In the next paragraphs we will see more specific examples.

2. Cox proportional hazards model

One of the most popular survival models is the Cox proportional hazards model (Cox, 1972):

$$\log \ h(t) = \alpha(t) + \beta x \qquad (1)$$

where, as before, *t* is the survival time, *h(t)* represents the hazard function, $\alpha(t)$ is the baseline hazard, $\beta$ is the slope parameter of the model and *x* is the regressor. The popularity of this model lies in the fact that it leaves the baseline hazard function $\alpha(t)$ (which we may alternatively designate as $\log h_0(t)$)
unspecified (no distribution assumed). It can be estimated iteratively by the method of partial likelihood of Cox (1972). The Cox proportional hazards model is semi-parametric because while the baseline hazard can take any form, the covariates enter the model linearly.

Cox (1972) showed that the $\beta$ coefficient can be estimated efficiently by the Cox partial likelihood function. Suppose that for each of a plurality of *K* subjects (labelled by $k$=1,...,$K$), we observe at corresponding time $t_k$ a certain nominal (i.e. yes/no) clinical event has occurred (e.g. whether there has been metastasis). This knowledge is denoted $e_k$. For example $e_k$ may be 0 if the event has not occurred by time $t_k$ (e.g. no tumour metastasis at time $t_k$) and 1 if the event has occurred (e.g. tumour metastasis at time $t_k$). Cox (1972) showed that the $\beta$ coefficient can be estimated efficiently by the Cox partial likelihood function, estimated as:

$$L(\beta_i) = \prod_{k=1}^{K} \left\{ \frac{\exp(\beta \ x_k)}{\sum_{j \in R(t_k)} \exp(\beta \ x_j)} \right\}^{e_k} \qquad (2)$$

where $R(t_k) = \{j: t_j \geq t_k\}$ is the risk set at time $t_k$. Typically, e is a binary variable taking value 0 = non-occurrence of the event until time *t* or 1 = occurrence of the event at time t. Later we will discuss a particular case of clinical event we consider in the work, without limiting though our model to this specific case.

The likelihood (2) is minimized by Newton-Raphson optimization method for finding successively better approximations to the zeroes (or roots) of a real-valued function, with a very simple elimination algorithm to invert and solve the simultaneous equations.

3. The good ness-of-split Measure of Survival and Selection of Prognostic Significant Genes

Assume a microarray experiment with $i$ = 1, 2, ..., $N$ genes, whose intensities are measured for $k$ = 1, 2, ..., $K$ breast cancer patients. The log-transformed intensities of gene *i* and patient *k* are denoted as $y_{i,k}$. Log-transformation serves for data "Gaussianization" and variance stabilization purposes, although other approaches, such as the log-linear hybrid transformation of Holder et al. (2001), the generalized logarithm transform of Durbin et al. (2002) and the data-driven Haar-Fisz transform have also been considered in the literature.

Associated with each patient *k* are a disease free survival time $t_k$ (in this work DFS time), a nominal clinical

event $e_k$ taking values 0 in the absence of an event until DFS time $t_k$ or 1 in the presence of the event at DFS time $t_k$ (DFS event) and a discrete gradual characteristic (histologic grade). Note that in this particular work the events correspond to the presence or absence of tumor metastasis for each of the *k* patients. Other types of events and/or survival times are possible to be analyzed by the model we will discuss below. Additional information, which is not utilized in this work, includes patients' age (continuous variable ranging from 28 to 93 years old), tumor size (in milimeters), breast cancer subtype (Basal, ERBB2, Luminal A, Luminal B, No subtype, normal-like), patients' ER status (ER+ and ER-) and distant metastasis (a binary variable indicating the presence or absence of distant metastasis).

Assuming, without loss of generality, that the *K* clinical outcomes are negatively correlated with the vector of expression signal intensity $y_i$ of gene *i,* patient *k* can be assigned to the high-risk or the low-risk group according to:

$$x_k^i = \begin{cases} 1 \ (high-risk), if \ y_{i,k} > c^i \\ 0 \ (low-risk), if \ y_{i,k} \leq c^i \end{cases} \quad (3)$$

where $c^i$ denotes the predefined cutoff of the *i*th gene's intensity level. In the case of positive correlation between the *K* clinical outcomes and $y_i$, patient *k* is simply assigned to one of the two groups according to:

$$x_k^i = \begin{cases} 1 \ (high-risk), if \ y_{i,k} \leq c^i \\ 0 \ (low-risk), if \ y_{i,k} > c^i \end{cases}$$

After specifying $x_k^i$, the DFS times and events are subsequently fitted to the patients' groups by the Cox proportional hazard regression model:

$$\log \ h_k^i(t_k | x_k^i, \beta_i) = \alpha_i(t_k) + \beta_i x_k^i \quad (4)$$

where, as before, $h^i_k$ is the hazard function and $\alpha_i(t_k) = \log h^i_0(t_k)$ represents the unspecified log-baseline hazard function for gene *i*; $\beta_i$ is the regression parameter to be estimated from the model; and $t_k$ is the patients' survival time.

To assess the ability of each gene to discriminate the patients into two distinct genetic classes, the Wald statistic (W) of the $\beta_i$ coefficient of model (4) is estimated by minimizing the univariate Cox partial likelihood function for each gene *i*:

$$L(\beta_i) = \prod_{k=1}^{K} \left\{ \frac{\exp(\beta_i^T x_k^i)}{\sum_{j \in R(t_k)} \exp(\beta_i^T x_j^i)} \right\}^{e_k}$$

where $R(t_k) = \{j: t_j \geq t_k\}$ is the risk set at time $t_k$ and $e_k$ is the clinical event at time $t_k$. The actual fitting of model (4) is conducted by the *survival* package in R (http://cran.r-project.org/web/packages/survival/index.html). The genes with the largest $\beta_i$ Wald statistics ($W_i$'s) or the lowest $\beta_i$ Wald *P* values are assumed to have better group discrimination ability and thus called *highly survival significant genes.* These genes are selected for further confirmatory analysis or for inclusion in a prospective gene signature set. Note that given $\beta_i$, one derives the Wald statistic, W, as:

$$W = \frac{\beta_i^2}{var(\beta_i)}$$

where $var(\beta_i) = \frac{1}{I(MLE)}$ and I denotes the Fisher information matrix of the $\beta_i$ parameter. Estimating the Wald *P* value, simply requires evaluation of the probability:

$$p - value = \Pr\left(\frac{\beta_i^2}{var(\beta_i)} > \chi_v^2\right)$$

where $\chi^2_v$ denotes the chi-square distribution with v degrees of freedom. Typically, v is the number of parameters of the Cox proportional hazards model and in our case v = 1. Expression (5) can be derived from the proper statistical tables of the chi-square distribution.

From Eqn. (3) notice that the selection of prognostic significant genes relies on the predefined cut-off value $c^i$ that separates the low-risk from the high-risk patients. The simplest cut-off basis is the mean of the individual gene expression values within samples, although other choices (e.g. median, trimmed mean, etc) could be also applied.

Summary of the invention

In general terms, a first aspect of the present invention proposes that, instead of testing the significance of the expression of individual genes individually, many pairs of genes are generated, and for each pair of genes clinical data is used to fit a statistical model to obtain the statistical significance of the ratio of the corresponding expression values. The clinical data characterizes for each of the patients the level of expressions of the genes and times until a clinical endpoint of interest ("survival time").

Thus, in contrast to the known techniques described above, reliance is not exclusively on linear (or sometimes non-linear) associations between the expression of individual genes and the clinical endpoint of interest. The present invention is motivated by the belief that it is biologically plausible that some genes may by themselves have no strong or obvious statistical correlation with survival, but when put together in a ratio with another gene, particularly one that it interacts with on a biological basis, could result in a "synergistic" correlation with outcome.

One possible expression of this aspect of the invention is a computerized method for identifying one or more pairs of genes, selected from a set of N genes, which are statistically associated with prognosis of a potentially fatal medical condition,

the method employing test data which, for each subject *k* of a set *of* $K^*$ subjects suffering from the medical condition, indicates (i) a survival time of subject *k*, and (ii) for each gene i, a corresponding gene expression value $y_{i,k}$ of subject *k*;
the method comprising:

(i) for each pair of a plurality of pairs of N genes (*i, j* with $i \neq j$):

(a) partitioning the $K^*$ subjects into two subsets according to whether $log(y_{i,k}) - log(y_{j,k})$ is respectively above or below a cut-off value $c_{i,j}$;
(b) computationally fitting the corresponding survival times of one of the subsets of the subjects to the Cox proportional hazard regression model, said fitting generating a regression parameter $\beta_{i,j}$; and
(c) obtaining from the regression parameter $\beta_{i,j}$ a significance value indicative of prognostic significance of the gene pair *i,j*; and

(ii) identifying one or more of the pairs of genes *i,j* for which the corresponding significance values have the highest prognostic significance.

Embodiments of the first aspect of the invention are defined by claims 2 to 10. The survival time may be an actual survival time (i.e. a time taken to die) or a time spent in a certain state associated with the medical condition, e.g. a time until metastasis of a cancer occurs.

Note that the K* subjects may be a subset of a larger dataset of K (K>K*) subjects. For example, the data for K* subjects can be used as training data, and the rest used for validation.

Alternatively, a plurality of subsets of the K subjects can be defined, and the method defined above is carried out independently for each of the subsets. Each of these subsets of the K subjects is a respective "cohort" of the subjects; if the cohorts do not overlap, they are independent training datasets. Note that each time the method is performed for a certain cohort, $K^*$ denotes the number of subjects in that cohort, which may be different from the number of subjects in other of the cohorts. After this, there is a step of discovering which pairs of genes were found to be significant for all the cohorts.

Optionally, there may be one of more further steps of reducing the number of candidate gene pairs, to find the most statistically significant.

Once one or more pairs of significant genes are identified, they can be used to obtain useful information in relation to a certain subject (typically not one of the cohort(s) of subjects) using a statistical model which takes as an input the ratio(s) of the expression values of the corresponding identified pair(s) of genes. The information may for example be susceptibility to the medical condition, the or prognosis (e.g. relating to recurrence or death) of a subject suffering from the condition.

Three specific gene pairs were identified using a method employing gene ratios in relation to breast cancer. These gene pairs are here referred to as:

(i) SEQ ID NO:1 and SEQ ID NO:2;
(ii) SEQ ID NO:3 and SEQ ID NO:4; and
(iii) SEQ ID NO:5 and SEQ ID NO:6.

The gene expression values of one or more of these three pairs of genes are obtained for a subject, the ratio(s) of the expression values of these pair(s) of genes are found, and then the results are entered into a statistical model which takes as an input the ratio(s) of the expression values of the pair(s) of genes, to obtain information about the subject in relation to breast cancer. The information may for example be susceptibility to the medical condition, or prognosis of a subject suffering from the condition.

Based on the same idea, an array may be found for obtaining the gene expressions of at least one (and preferably all) of the three pairs of genes (i)-(iii). Since these three pairs of genes are known to have statistical significance in relation to breast cancer, the array need not additionally be designed to obtain the expression values for a great many other genes, and therefore the array can be manufactured at lower cost than conventional arrays which measure the expression values of hundreds or thousands of other genes. In fact, preferably the present arrays may measure the expression values of no more than 100 genes, or more preferably no more than 20 genes, or even just the three pairs of genes (i) to (iii).

The method, as herein described may be performed with a computer system and/or apparatus. The invention also proposes computer program products (e.g. stored on a tangible recording medium) which are software operable by a computer to cause the computer to perform the computational steps of the method.

Having now generally described the invention, the same will be more readily understood through reference to the following exemplary embodiments which are provided by way of illustration, and are not intended to be limiting of the present invention.

Brief description of the figures

Fig. 1 shows the steps of a first method according to the invention;

Fig. 2 shows the steps of a second method according to the invention;

Fig. 3 illustrates gene expression batch effects and normalizing properties of ratios in the second embodiment;

Fig. 4 illustrates the sum of weights and prognostic group assignment for a 3-ratio model derived in the second embodiment;

Fig. 5, which is composed of Fig. 5A and 5B, illustrates training and test results of a 3-ratio predictor based on the 3-ratio model; and

Figure 6, which is composed of Fig. 6A and 6B, illustrates the Prognostic potential of the 3-ratio predictor using a different subject cohort.

Definitions

"Array" or "microarray," as used herein, comprises a surface with an array, preferably an ordered array, of putative binding (e.g., by hybridization) sites for a sample which often has undetermined characteristics. An array can provide a medium for matching known and unknown nucleic acid molecules based on base-pairing rules and automating the process of identifying the unknowns. An array experiment can make use of common assay systems such as microplates or standard blotting membranes, and can be worked manually, or make use of robotics to deposit the sample. The array can be a macro-array (containing nucleic acid spots of about 250 microns or larger) or a micro-array (typically containing nucleic acid spots of less than about 250 microns).

The term "cut-off" represented by *c* in the context of the classification, refers to a value for which if the expression level of a particular nucleic acid molecule (or gene) in a subject is above the cut-off, the subject is classified into a first classification group; and if the expression level of the nucleic acid molecule is below or equal to the cut-off, the subject is classified into a second classification group.

The term "gene" refers to a nucleic acid molecule that encodes, and/or expresses in a detectable manner, a discrete product, whether RNA or protein. It is appreciated that more than one nucleic acid molecule may be capable of encoding a discrete product. The term includes alleles and polymorphisms of a gene that encodes the same product or an analog thereof.

Detailed description of the invention

Standard molecular biology techniques known in the art and not specifically described were generally followed as described in Sambrook and Russel, Molecular Cloning: A Laboratory Manual, Cold Springs Harbor Laboratory, New York (2001).

First embodiment

The steps of the method are shown in Fig. 1. The method is carried out using data which, for one or more cohorts of subjects, gives the expression values within tumours of for a large number of genes. For example, it may be carried out using the Affymetrix U133A and B Genechips which consist of about 45,000 probes. A first step of the method (step 1) is to generate a number of pairs of genes. All possible combinations of these genes exceeds 5,000,000 possible combinations.

Label the subjects of one cohort k = 1, 2, ...., $K^*$. For each subject $k$, there is an expression value $y_{i,k}$ of each of the genes $i$, and the logarithm of this expression value is given by log $y_{i,k}$. For each gene and for each of the indentified probes, we stratify the subjects (step 2) in the following way. For each gene $i$ and each pair of genes ($i, j$ with $i \neq j$) we define a cut-off parameter $c_i$ or $c_{i,j}$ respectively, and define:

$$x^i{}_k = \begin{cases} 1 \text{ , if } y_{i,k} > c^i \\ 0 \text{ , if } y_{i,k} \le c^i \end{cases} \quad \text{and} \quad x^{i,j}{}_k = \begin{cases} 1 \text{ , if } \log y_{i,k} - \log y_{j,k} > c^{i,j} \\ 0 \text{ , if } \log y_{i,k} - \log y_{j,k} \le c^{i,j} \end{cases} \qquad (5)$$

Due to the mathematical identity log A-log B=log(A/B) for any A and B, the cut-off determines $x^{i,j}{}_k$ according to the ratio of the expression levels $y_{i,k}$ and $y_{j,k}$.

The values of $c_i$ and $c_{i,j}$ may be selected to according to a mean or a median over the cohort.

The significance of each individual gene is then obtained by fitting the survival data to Eqn. (4) above (step 3). The significance of each pair of genes $i,j$ may be determined by fitting the survival data to:

$$\log h^{i,j}{}_k(t_k \mid x_k^{i,j}, \beta_{i,j}) = \alpha_i(t_k) + \beta_{i,j} \cdot x_k^{i,j}, \qquad (6)$$

We then assess the significance of genes and pairs of genes from the values of $\beta_i$ and $\beta_{i,j}$ respectively, for example by obtaining p values from a likelihood ratio test or log rank test. The result reflects the prognostic value of a given gene or gene ratio. The more significant the p-value, the greater the gene or gene ratio is associated (in a linear manner) with patient survival. Thus significant pairs of genes, or individual genes, are identified (step 4).

The method of Fig. 1 is performed for multiple comparable clinical patient cohorts, to discriminate true (reproducible) prognostic associations from those resulting from false discovery. That is, we identify the genes and gene pairs which are independently found to be significant for all of the cohorts. Then, in a final independent validation cohort, the prognostic performance of the selected genes or gene ratios is independently evaluated. The intention of this methodology is to identify robust prognostic expression ratios that exceed the prognostic performance of individual genes, and that thus can be used alone or in combination with each other, as well as any other biomarker or other clinical prognostic variable or marker, to enhance patient prognosis.

To test the theory, we targeted a specific clinical entity: early stage (lymph node negative, small tumor diameter) breast cancer. The rationale is that multiple independent cohorts, where the tumor material has been assessed by expression microarrays (namely the Affymatrix U133 platform), and where long term clinical follow-up information is available (i.e. distant metastatis-free survival data), exist and can be utilized for the validation of the methodology. We seek to determine if the gene expression ratios can significantly enhance patient prognosis in this disease type over convention and widely publicized methods involving single gene measurements.

Once significant gene ratios (and optionally also genes) have been identified, they can be used to obtain information relating to specific subjects (e.g. prognosis for those subjects) using measured expression values of those genes in those subjects. This can be done by forming a model (e.g. a Cox proportional hazards model) in which those gene ratios are weighted with values obtained based on a training dataset, and extracting the information from the model, e.g. in the form of diagram indicating survival probability for the subject against time.

Another aspect of this example relates to the biological inferences that can be drawn through further analysis of the "best" prognostic expression ratios. In some instances we expect that the prognostic significance of the gene ratios lies in the ratio's capacity to provide better functional information regarding pathway activity, or other conditional relationships that drive aggressive tumor growth, than individual gene expression levels. Thus, the methodology may lead to the discovery of clinically relevant biological/pathological interactions that could ultimately be therapeutically targetable. Examples of such interactions could be transcription factors/targets, phosphatases/kinases, agonists/antagonists of the same pathway, oncogenes/tumor suppressor genes, etc.

Second embodiment

We now present experimental results obtained using 5 cohorts of subjects, referred to here as the Uppsala, Erasmus, Oxford, Stockholm and Transbig cohorts, in a method which is a second embodiment of the invention and illustrated in Fig. 2.

Details of the Erasmus cohort are given in Wang et al. Details of the Oxford cohort are given in Loi et al. Details of the Transbig cohort are given in Desmedt et al.

1. Cohort Selection and processing of micro-array data

This experiment has focused on the clinical entity: *LN-, untreated breast cancer.* Representing this entity is a large collection of patient data we have assembled that comprises 5 independent patient cohorts totaling 708 cases for which we have corresponding gene expression profiles and clinical annotation. The primary clinical outcome studied was distant metastasis-free survival (i.e., the time interval from initial diagnosis of the primary tumor to distant recurrence, or last follow-up whereby the patient was diagnosed as relapse-free) within a 10-year window.

Each cohort was selected based on compliance with the following 2 clinical criteria: 1) median patient follow-up of at least 5 years, and 2) all patients were treated by surgery with/without local radiation, and without systemic adjuvant (or neo-adjuvant) therapy. Our rationale for selecting node-negative, untreated patients is two-fold. First, that these patients have received no systemic adjuvant therapy ensures that the prognostic associations we discover are not confounded by systemic treatment effects. Second, this subgroup represents early stage breast cancer - for which the decision to treat or not to treat (or how aggressively to treat) is controversial and thus would benefit from better prognostic markers. Clinical characteristics of the cohorts and corresponding reference information are shown in Table 1.

Table 1. Clinical and reference information for Patient Subgroup #1.

| Cohort Name | Systemic Therapy | Training/ Validation | # of Patients | Median Follow-Up (years) | %-age 10-yr Recurrence | GEO Accession | Literature Reference |
|---|---|---|---|---|---|---|---|
| Uppsala | none | training | 118 | 10.3 | 23.7 | GSE3 494 | Miller, et al. (1) |
| Stockholm | none | training | 37 | 5.9 | 51.4 | GSE1 456 | Pawitan, et al. (2) |
| OXFU | none | training | 69 | 8.7 | 33.3 | GSE6 532 | Loi, et al. (3) |
| EMC | none | training | 286 | 7.2 | 37.4 | GSE2 034 | Wang, et al. (4) |
| TBIG | none | validation | 198 | 12.0 | 25.3 | GSE7 390 | Desmedt, et al. (5) |

All patient/tumor clinical data were obtained from original published reports or by personal communication with the study leaders. All raw microarray data were downloaded from the NCBI's Gene Expression Omnibus (GEO) via the GEO accession numbers presented in Table 1. All five microarray studies were conducted on the Affymetrix U133A or U133 Plus 2.0 array platforms which contain 22,268 overlapping gene probe sets. Each of the 708 gene array profiles was evaluated for quality according to our previously published methods (Ploner, 2005). All expression profiles were MAS5.0 processed, scaled to a median target intensity of 500 and $\log_2$ transformed. (Post-scaling intensity values <10 were adjusted to 10 in order to prevent negative values after log transformation.) For discovery and validation purposes, the cohorts were divided into training (Uppsala, Stockholm, OXFU, and EMC) and test (TBIG) cohorts.

2. Data formatting and parallel computing

Prior to computing the gene ratios, the Affymetrix data were filtered to exclude control probe sets, probe sets that failed to map to human gene sequences with high specificity(Vlad), and probe sets with mean signal intensities of 50 units or less in 2 or more training cohorts. After exclusions, 18,190 probe sets remained for ratio analysis. All possible pair-wise combinations of these probe sets equalled $18190^2/2$, or 165,438,050 unique gene-pair combinations (step 11 of Fig. 2).

We next analyzed all unique gene-pair combinations, independently, in each of the 4 training cohorts, for statistical correlations with DMFS by Cox Proportional-Hazards Regression (step 12 of Fig. 2). Importantly, standard computing strategies that involve one or several high-performance PCs is insufficient for the timely computation of over a half billion Cox regressions due to physical and virtual memory constraints. Thus, we performed our analysis on Singapore's most powerful super computer. Called the Hilbert Cluster, this system comprises of 500 AMD Opteron processors and can be freely accessed by researchers at the Genome Institute of Singapore. Using a parallel computing program, we computed the hazard ratios and p-values for ratios representing all gene-pair combinations in each of the 4 training cohorts (i.e., 662 million calculations).

3. Selection of top gene ratios for model building

Using a data extraction script, the results files output from the Hilbert Cluster were filtered to retain the gene ratios having the following properties: 1) p-value >10-fold more significant than either of the 2 contributing genes (in at least 1 of 4 cohorts), 2) p-value <0.01 in all 4 training cohorts, and 3) consistent directionality (i.e., the ratio must be either positively or negatively correlated with DMFS in all 4 cohorts). In total, we identified 5284 ratios that passed these criteria (step 13 of Fig. 2).

4. Training and testing of prognostic models.

To develop prognostic models from the selected gene ratios described above, we first combined all 510 tumor (ratio) profiles from the 4 initial training cohorts into a single "unified" training cohort (step 14 of Fig. 2). Interestingly, the mathematical challenge of combining multiple cohorts into one unified cohort was facilitated by an intrinsic property of gene ratios. In microarray studies, factors that generate artificial signals (noise) in the data (e.g. inconsistent RNA handling procedures, variation in array/reagent batches, fluctuations in scanner laser power, etc) are frequently accounted for and controlled. However, subtle methodological differences *between* studies can give rise to noise producing "batch effects" that can be observed when identically-processed microarray data from different studies are compared. Batch effects can be recognized by examining the expression distributions of individual genes across studies.

Figure 3 illustrates gene expression batch effects and normalizing properties of ratios. Left and middle panels show the inequality of expression distributions of two randomly-selected Affymetrix probesets compared across patient cohorts. The $\log_2$ intensities of the two probe sets were converted to ratios, and the resulting "normalized" ratio distributions are shown in the right panel. As shown below in Figure 3 (left and middle panels), the expression distribution for a given probe set can vary widely across microarray cohorts as the result of batch effects.

However, we have observed that this variation is largely systematic, affecting all gene distributions within a cohort in a similar way. This property is illustrated in the left and middle panels of Figure 3, where two distinct probe sets, representing high and intermediate expression ranges, display distribution differences that are *conserved between cohorts* in a relative manner. Because these effects are systematic, transforming the data into gene ratios has a cross-cohort normalizing effect that can be seen in the right panel of Figure 3, whereby the median ratio within cohorts shows <5% variation across cohorts. Thus, converting expression data to ratios serves to correct cross-cohort batch effects. This batch-correction property of ratios has important implications in model training and testing, as this process operates on the assumption that the distribution pattern of a given linear covariate (e.g., a ratio) is comparable across cohorts.

As our goal was to identify a small number of gene pairs that contribute to prognosis in a synergistic fashion (step 15 of Fig. 2), we developed prognostic models using an ensemble learning algorithm called AdaBoost. Adaboost chooses a collection of features (ie, gene ratios) that can collectively discriminate between classes, for example non-recurrent (-1's) and recurrent cases (1's), in a training set. The process of selecting ratios into the model is iterative, beginning with the ratio that best separates the recurrent and non-recurrent cases. For this "best separator", a single ratio threshold is identified for the optimal dichotomy of recurrent and non-recurrent cases, and a prognostic weight that reflects the predicted class (positive for 1's, negative for - 1's) is then applied to each case. Then, the algorithm identifies (from the remaining ratios) the ratio that best complements the ratio that was chosen first. This is done to optimize a scoring function that can be thought of as rewarding the algorithm for making more correct classifications, and also for making progress on cases that were initially misclassified. The third ratio is then chosen based on its ability to complement the first two, and so on. Cases are ultimately classified as "recurrent" or "non-recurrent" by taking a weighted vote over the thresholds computed for each of the chosen ratios. The sign (+/-) of the sum of the weights (SOW) reflects the predicted class of a case, and the magnitude of the SOW reflects the confidence in that prediction. Thus, the SOWs can be viewed as prognostic classes for separating cases (like a recurrence score), where the number of classes is equal to $2^x$; where *x* equals the number of ratios in the model.

Typically, in analogous classification procedures, the ideal prognostic model is extrapolated from a feature selection/internal cross-validation strategy to minimize data overfitting and to determine the optimal number of features for inclusion in the model. In our case, however, we used a clinically-guide approach to select a minimal number of ratios for inclusion and to simultaneously define thresholds for "good", "intermediate" and "poor" outcome categories. Figure 4 shows the sum of weights and prognostic group assignment for the 3-ratio model. The 3-ratio model applied to the 510 cases of the training cohort gave rise to eight SOW classes from which 3 prognostic groups were defined based on outcome criteria.

Specifically, we asked at what number of ratios in the model can we identify one or more SOW classes that correspond to patients with a distant recurrence rate of <10% at 10 years. The answer, we observed, was 3 or more ratios. With only 3 ratios in the model, the SOW of greatest negative magnitude (-0.86) contained 149 cases which together had a 10-year recurrence rate of only 8.4% - which we subsequently defined as the good outcome group. We then classified the remainder of cases into intermediate and poor outcome groups using SOW thresholds that provided roughly equivalent survival curve separation between the three groups. This can be seen in the Kaplan-Meier plot of Figure 5A. Shown are survival curves for predicted low, intermediate and poor outcome groups in the training (A) and test (B) cohorts.

To test the prognostic performance of the 3-ratio model in the TBIG cohort, we extracted the expression intensities of the 3 gene pairs, computed their corresponding ratios, and applied the Adaboost thresholds and weights as defined in the model. The resulting good, intermediate and poor outcome groups showed significantly different survival rates (p<0.0001; likelihood ratio test) with the good outcome group having a 10-year recurrence rate of 7.8% - comparable to the training set (Figure 5B). Notably, in this cohort, while the intermediate and poor outcome groups faired significantly worse than the good outcome group, the rates of recurrence in these groups was less than that observed in the training set, perhaps due to some degree of overfitting, residual batch-effect noise or phenotypic differences between the two cohorts.

With an interest in the reproducibility of each ratio's contribution to the performance of the model, we examined the prognostic potential of each ratio in the TBIG cohort (Table 2). By univariate analysis, we found that all three ratios were significantly correlated with distant recurrence. Moreover, in a multivariate model, each ratio remained significant, suggesting a unique contribution from each ratio to the prognostic power of the 3-ratio predictor.

Table 2. Univariate and multivariate analysis of ratios by Cox regression.

| **TBIG Cohort (198 LN-, untreated)** | **Univariate** | | **Multivariate** | |
|---|---|---|---|---|
| **Variables** | **Hazard Ratio (95% CI)** | ***P*-value** | **Hazard Ratio (95% CI)** | ***P*-value** |
| RATIO #1 | 2.89 (1.48-5.66) | 0.002 | 2.42 (1.22-4.76) | 0.01 |
| RATIO #2 | 2.35 (1.29-4.25) | 0.005 | 1.96 (1.06-3.61) | 0.03 |
| RATIO #3 | 2.20 (1.25-3.87) | 0.006 | 1.73 (0.97-3.12) | 0.06 |

5. Comparison of the 3-ratio predictor with conventional variables.

A primary reason for selecting the TBIG cohort for model testing was its detailed annotation for conventional prognostic markers (including the test results for the 70-gene MammaPrint assay) with which we could compare the prognostic value of our predictor via multivariate analysis. In this cohort, the 3-ratio predictor, tumor size, grade, and ER status were all significantly correlated with DMFS (Table 3). However, when considered in the multivariate model, only the 3-ratio predictor and tumor size remained significant at the 0.05 level, reflecting their unique contributions to prognosis. Furthermore, when considered in the presence of the Mammaprint score, the 3-ratio predictor remained significant, indicating an additive prognostic contribution unique from that of MammaPrint.

Table 3. Univariate and multivariate analysis of the 3-ratio predictor and conventional markers.

| TBIG Cohort (198 LN-, untreated) | Univariate | | Multivariate | |
|---|---|---|---|---|
| **Variables** | **Hazard Ratio (95% CI)** | ***P*-value** | **Hazard Ratio (95% CI)** | ***P*-value** |
| 3-Ratio Score (1, 2, 3) | 2.32 (1.49-3.62) | 0.0004 | 2.08 (1.27-3.40) | 0.004 |
| Histologic Grade (1, 2, 3) | 1.57 (1.02-2.40) | 0.04 | 0.85 (0.50-1.43) | 0.54 |
| Tumor size (per 1-cm increment) | 1.57 (1.18-2.08) | 0.002 | 1.36 (1.01-1.83) | 0.04 |
| Patient age (per 10-yr increment) | 1.02 (0.71-1.48) | 0.9 | 1.02 (0.70-1.47) | 0.92 |
| ER status (+, -) | 0.43 (0.25-0.75) | 0.003 | 0.53 (0.27-1.02) | 0.06 |
| LN status (all negative) | NA | NA | NA | NA |
| | | | | |
| 3-Ratio Score (1, 2, 3) | 2.32 (1.49-3.62) | 0.0004 | 1.67 (1.01-2.70) | 0.04 |
| MammaPrint Score (0,1) | 7.17 (2.58-19.9) | 0.0002 | 5.46 (1.90-15.7) | 0.002 |

Note that in this embodiment, there is no pre-selection of genes based on survival/prognostic association. Rather, all gene pairs (i.e. ratios) are considered by Cox regression. So the embodiment screens all possible gene combinations (limited only by the number of probes on the microarray) looking for those ratios with the greatest robustness (i.e. reproducible survival associations across 4 independent training datasets, in our example), then we combined all these ratios together into one set, and combined all the tumour samples from the 4 training datasets into one dataset, and asked Adaboost to find the few ratios with the greatest complementarity in predicting outcome (i.e. that work well together in a prognostic model).

5. identity of top three Gene Pairs

Pair #1, Gene #1:

Genbank sequence: NM_006472 (SEQ ID NO: 1)

Affymetrix ID: 201010_s_at
Name: Thioredoxin interacting protein; TXNIP (symbol)
Aliases: VDUP1; Thioredoxin-binding protein 2; Vitamin D3-upregulated protein 1

Pair #1, Gene #2

Genbank sequence: AI826060 (SEQ ID NO: 2)

Affymetrix ID: 202069_s_at
Name: Isocitrate dehydrogenase 3 (NAD+)alpha: IDH3A (symbol)
Aliases: Isocitrate dehydrogenase 3, alpha subunit

Pair #2, Gene #1

Genbank sequence: NM_002497 (SEQ ID NO: 3)

Affymetrix ID: 20464_at
Name: NIMA (never in mitosis gene a)-related kinase 2; NEK2 (symbol)
Aliases: NIMA-related kinase 2

Pair #2, Gene #2

Genbank sequence: T15766 (SEQ ID NO: 4)

Affymetrix ID: 213276_at

Name: Calcium/calmodulin-dependent protein kinase (CaM kinase) II beta; CAMK2B (symbol)
Aliases: 2.7.1.123; CAM2; CAMKB; MGC29528; CaM kinase II beta subunit; CaM-kinase II beta chain; proline rich calmodulin-dependent protein kinase

Pair#3, Gene #1

Genbank sequence: NM_005008 (SEQ ID NO: 5)

Affymetrix ID: 201076_at
Name: NHP2 non-histone chromosome protein 2-like 1 (S. cerevisiae); NHP2L1 (symbol)
Aliases: NHP2-like protein 1; Non-histone chromosome protein 2, S.cerevisiae, homolog-like 1; U2/U6-15.5K protein

Pair#3, Gene #2

Genbank sequence NM_022341 (SEQ ID NO: 6)

Affymetrix ID: 219575_s_at
Name: Peptide deformylase (mitochondrial); COG8 (symbol)
Aliases: Transcribed locus, strongly similar to NP_115758.3 component of oligomeric golgi complex 8 [Homo sapiens].

6. Validation data

For a final assessment of ratio performance, we analyzed another well known breast cancer microarray cohort from the Netherlands Cancer Institute (NKI). The NKI cohort is comprised of 295 consecutive breast cancer patients with detailed clinical annotation, and the patient samples were profiled on a comprehensive Agilent microarray. Notably, microarray analysis of this cohort enabled the discovery and validation of the 70-gene MammaPrint signature. While the Affymetrix and Agilent microarrays share the ability to detect a large common set of overlapping genes, the probe design algorithms are different, resulting in oligonucleotides of different sequence and length otherwise designed to detect the same genes. Upon accessing this microarray dataset(), we found that each of our 6 3-ratio predictor genes was represented by at least 1 probe on the Agilent array. In the case of redundant probes, we averaged the expression values to obtain a single value for each gene. Next, we computed the gene ratios, then divided the cohort into 2 subgroups: LN-, untreated (n=141) and LN+ (n=144, >80% were treated with chemotherapy, hormone therapy, or both). Due to technical differences between the array platforms than influence ratio distributions, the 3 ratios were "retrained" by the Adaboost algorithm to identify appropriate thresholds and weights for each ratio. In effect, this optimizes the survival separation achievable by the 3 ratios (and thus could be susceptible to overfitting). While this does not constitute an independent validation of the predictor's performance, it does allow us to evaluate the relative prognostic potential of the 3-ratio predictor in the context of a different gene expression platform (Agilent) and LN+, systemically-treated patients. First, by univariate analysis, we found that each ratio was significantly associated with DMFS by Cox regression in the NKI cohort (p-values: 0.02, 0.006, 0.002, for ratios #1, #2 and #3, respectively). Next, by Kaplan-Meier analysis, we observed a very significant separation between the good, intermediate and poor outcome groups (Figure 6A) comparable to that observed in the original training and test (TBIG) cohorts.

Using the thresholds and weights determined in the LN- cohort, we then directly tested the 3-ratio predictor on the LN+ NKI cohort. As can be seen in Figure 6B, the good outcome group maintains a good prognosis (especially for the first 6 years) while the intermediate and poor outcome group survival rates are similar to one another, mimicking that of the LN- intermediate group shown in Figure 6A. As the majority of patients in the LN+ poor outcome group were systemically treated, the improved outcome in this group (despite the worse prognosis conferred by positive lymph nodes) may indicate a therapeutic benefit for these patients. However, more thorough treatment-control parameters and larger datasets will be needed to ascertain the prognostic and clinical value of gene ratio predictors in the adjuvant setting (this issue is further addressed in the Research Design and Methods section). Together, our observations in the NKI cohort demonstrate the general robustness of the prognostic power of the gene ratios in the context of a different gene-expression detection platform and systemically treated patients.

References

The disclosure of the following documents is hereby incorporated by reference:

Breslow, N.E., "Covariance analysis of censored survival data", Biometrics, vol. 30, pp 89-99, 1974.

Cox, D.R. and Snell, E.J., "A general definition of residuals (with discussion)", Journal of the Royal Statistical Society, Series B, vol. 30, pp 248-265, 1968.

Cox R.D. and Oakes, D., Analysis of Survival Data. London: Chapman and Hall, 1984.

Desmedt C, Piette F, Loi S, Wang Y, Lallemand F, Haibe-Kains B, Viale G, Delorenzi M, Zhang Y, d'Assignies MS, Bergh J, Lidereau R, Ellis P, Harris AL, Klijn JG, Foekens JA, Cardoso F, Piccart MJ, Buyse M, Sotiriou C; TRANSBIG Consortium. Strong time dependence of the 76-gene prognostic signature for node-negative breast cancer patients in the TRANSBIG multicenter independent validation series. Clin Cancer Res 2007 Jun 1;13(11):3207-14.

Efron, B. and Tibshirani, R.J., An Introduction to the Bootstrap. New York: Chapman and Hall, 1994.

Ivshina, A.V., George, J., Senko, O et al, "Genetic reclassification of histologic grade delineates new clinical subtypes of breast cancer", Cancer Research, vol. 66, pp 10292-10301, 2006.

Kaplan, E. L. and Meier, P., "Nonparametric estimation from incomplete observations". JASA, vol. 53, 457-48, 1958.

Kuznetsov, V.A., Senko, O.V., Miller, L.D. and Ivshina, A., "Statistically Weighted Voting Analysis of Microarrays for Molecular Pattern Selection and Discovery Cancer Genotypes", International Journal of Computer Science and Network Security, vol. 6, pp 73-83, 2006.

Loi S, Haibe-Kains B, Desmedt C, Lallemand F, Tutt AM, Gillet C, Ellis P, Harris A, Bergh J, Foekens JA, Klijn JG, Larsimont D, Buyse M, Bontempi G, Delorenzi M, Piccart MJ, Sotiriou C. Definition of clinically distinct molecular subtypes in estrogen receptor-positive breast carcinomas through genomic grade. J Clin Oncol. 2007 Apr 1;25(10):1239-46.

Loughin, T.M., "A residual bootstrap for regression parameters in proportional hazards model", J. of Statistical and Computational Simulations, vol. 52, pp 367-384, 1995.

Motakis, E., Nason, G.P., Fryzlewicz, P. and Rutter, G.A., "Variance stabilization and normalization for one-color microarray data using a data-driven multiscale approach", Bioinformatics, vol. 22, pp 2547-2553, 2006.

Millenaar, F. F., Okyere, J., May, S.T., van Zanten, M., Voesenek, L.A.C.J. and Peters, A.J.M., "How to decide? Different methods of calculating gene expression from short oligonucleotide array data will give different results", BMC Bioinformatics, vol. 7, no. 137, 2006.

Pawitan, Y., Bjohle, J., Amler, L., at al, "Gene expression profiling spares early breast cancer patients from adjuvant therapy: derived and validated in two population-based cohorts", Breast Cancer Research, vol. 7, pp R953-R964, 2005.

Wang Y, Klijn JG, Zhang Y, Sieuwerts AM, Look MP, Yang F, Talantov D, Timmermans M, Meijer-van Gelder ME, Yu J, Jatkoe T, Berns EM, Atkins D, Foekens JA. Gene-expression profiles to predict distant metastasis of lymphnode-negative primary breast cancer. Lancet. 2005 Feb 19-25;365(9460):671-9.

## Claims

1. A computerized method for identifying one or more pairs of genes, selected from a set of N genes, which are statistically associated with prognosis of a potentially fatal medical condition,
the method employing test data which, for each subject *k* of a set *of* $K^*$ subjects suffering from the medical condition, indicates (i) a survival time of subject *k*, and (ii) for each gene *i*, a corresponding gene expression value $y_{i,k}$ of subject *k*; the method comprising:

   (i) for each pair of a plurality of pairs of the N genes ($i$, $j$ with $i \neq j$):

      (a) partitioning (2) the $K^*$ subjects into two subsets according to whether $log(y_{i,k})$ - $log(y_{j,k})$ is respectively above or below a cut-off value $c_{i,j}$;
      (b) computationally (3; 12) fitting the corresponding survival times of one of the subsets of the subjects to the Cox proportional hazard regression model, said fitting generating a respective regression parameter $\beta_{i,j}$; and

(c) obtaining from the regression parameter $\beta_{i,j}$ a significance value indicative of prognostic significance of the gene pair *i,j*; and

(ii) identifying (4; 13) one or more of the pairs of genes *i,j* for which the corresponding significance values have the highest prognostic significance.

**2.** A computerized method for discovering one or more pair of genes, selected from a set of N genes, which are statistically associated with prognosis of a potentially fatal medical condition, the method including:

(i) for each of a plurality of sets of subjects, identifying a respective set of gene pairs from the set of N genes by a method according to claim 1; and
(ii) discovering (13) one or more gene pairs which are in common to the plurality of sets of gene pairs.

**3.** A computerized method according to claim 2 further including verifying the prognostic significance of the discovered gene pairs using data describing the survival times and corresponding gene expression levels of a further set of subjects.

**4.** A computerized method according to claim 2 further including applying a boosting algorithm (15) to select one or more pairs of gene pairs from the gene pairs which are in common to the plurality of sets of gene pairs.

**5.** A computerized method according to claim 1 wherein the medical condition is cancer, and the expression levels are from samples of respective tumours in the $K^*$ subjects.

**6.** A method according to claim 5 wherein the medical condition is breast cancer.

**7.** A method according to claim 1 in which the survival time is survival time until mortality.

**8.** A method according to claim 1 in which the survival time is a survival time without metastasis of a cancer.

**9.** A computerized method of obtaining information about a first subject in relation to a potentially-fatal medical condition, the method including:

(i) identifying, by a method according to claim 1, from among a set of N genes, one or more pairs of genes which are statistically associated with prognosis of the medical condition;
(ii) for each of the one or more identified pairs of genes *i, j* obtaining corresponding gene expression values $y_i$ and $y_j$ of the first subject; and
(iii) obtaining said information using a ratio of the obtained gene expression values.

**10.** A computerized method according to claim 9 in which said information is a prognosis for the first subject who is suffering from the medical condition, a susceptibility of the first subject to the medical condition, a prediction of the recurrence of the medical condition, or a recommended treatment for the medical condition.

**11.** A computer system arranged to perform a method according to any of claims 1 to 10.

**12.** A computer program product comprising software operable by a computer to cause the computer to perform a method according to any of claims 1 to 10.

## Patentansprüche

**1.** Computergestütztes Verfahren zum Identifizieren von einem oder mehreren Paaren von Genen, die ausgewählt sind aus einer Reihe von N Genen, die statistisch mit einer Prognose einer potenziell tödlichen Erkrankung verbunden sind,
wobei in dem Verfahren Testdaten eingesetzt werden, die bei jedem Patienten *k* einer Reihe von $K^*$ Patienten, die an dem Krankheitsbild leiden, anzeigt: (i) eine Überlebenszeit des Patienten *k* und (ii) für jedes Gen *i* einen entsprechenden Wert der Expression des Gens $y_{i,k}$ des Patienten *k*;
wobei das Verfahren umfasst:

(i) für jedes Paar eine Mehrzahl von Paaren der N Gene (i, j mit $i \neq j$):

(a) Unterteilung (2) der $K^*$ Patienten in zwei Unterreihen danach, ob $\log(y_{i,k})$ - $\log(y_{l,k})$ jeweils oberhalb oder unterhalb eines Schwellwertes $c_{i,j}$ ist;
(b) rechnerisch Ausgleichen (3 ; 12) der entsprechenden Überlebenszeiten einer der Unterreihen der Patienten zu dem Regressionsmodell des "Proportional Hazard" nach Cox, wobei das besagte Ausgleichen einen entsprechenden Regressionsparameter $\beta_{i,j}$ erzeugt; und
(c) aus dem Regressionsparameter $\beta_{i,j}$ einen signifikanten Wert erhalten, der kennzeichnend ist für die prognostische Signifikanz des Genpaares *i,j*; und

(ii) Identifizieren (4, 13) von einem oder mehreren Paaren von Genen *i,j*, bei denen die entsprechenden Werte der Signifikanz die höchste prognostische Signifikanz habe.

**2.** Computergestütztes Verfahren zum Auffinden von einem oder mehreren Paaren von Genen, die ausgewählt sind aus einer Reihe von N Genen, die statistisch mit einer Prognose einer potenziell tödlichen Erkrankung verbunden sind, wobei das Verfahren einschließt:

(i) für jede einer Mehrzahl von Reihen von Patienten das Identifizieren einer entsprechenden Reihe von Genpaaren aus der Reihe von N Genen mit Hilfe eines Verfahrens nach Anspruch 1, und
(ii) Auffinden (13) von einem oder mehreren Genpaaren, die der Mehrzahl von Reihen von Genpaaren gemeinsam sind.

**3.** Computergestütztes Verfahren nach Anspruch 2, ferner einschließend das Verifizieren der prognostischen Signifikanz der aufgefundenen Genpaare, indem Daten verwendet werden, die die Überlebenszeiten und entsprechenden Werte der Genexpression einer weiteren Reihe von Patienten beschreiben.

**4.** Computergestütztes Verfahren nach Anspruch 2, ferner einschließend das das Anwenden eines Boosting Algorithmus (15), um ein oder mehrere Paare von Genen aus den Genpaaren zu selektieren, die der Mehrzahl von Reihen von Genpaaren gemeinsam sind.

**5.** Computergestütztes Verfahren nach Anspruch 1, wobei die Erkrankung Krebs ist und die Werte der Expression von Proben entsprechender Tumore in den $K^*$-Patienten stammen.

**6.** Verfahren nach Anspruch 5, wobei die Erkrankung Brustkrebs ist.

**7.** Verfahren nach Anspruch 1, bei welchem die Überlebenszeit die Überlebenszeit bis zur Mortalität ist.

**8.** Verfahren nach Anspruch 1, bei welchem die Überlebenszeit eine Überlebenszeit ohne Metastasen eines Krebses ist.

**9.** Computergestütztes Verfahren zum Erhalten von Information über einen ersten Patienten in Bezug auf eine potenziell tödliche Erkrankung, wobei das Verfahren einschließt:

(i) Identifizieren mit Hilfe eines Verfahren nach Anspruch 1 aus einer Reihe von N Genen ein oder mehrere Paare von Genen, die mit der Prognose der Erkrankung statistisch verbunden sind;
(ii) für jedes des einen oder der mehreren Paare von Genen *i, j* entsprechende Werte der Genexpression $y_i$ und $y_j$ von dem ersten Patienten erhalten;
(iii) Erhalten dieser Information, indem ein Quotient der erhaltenen Werte der Genexpression verwendet wird.

**10.** Computergestütztes Verfahren nach Anspruch 9, wobei die Information eine Prognose für den ersten Patienten ist, der an der Erkrankung leidet, für eine Anfälligkeit des ersten Patienten für diese Erkrankung, eine Vorhersage ist für das Wiederauftreten der Erkrankung oder für eine empfohlene Behandlung für die Erkrankung.

**11.** Computersystem, ausgelegt zur Ausführung des Verfahrens nach einem der Ansprüche 1 bis 10.

**12.** Erzeugnis eines Computerprogramms, umfassend Software, die mit Hilfe eines Computers betriebsfähig ist, um den Computer zur Ausführung des Verfahrens nach einem der Ansprüche 1 bis 10 zu veranlassen.

**Revendications**

1. Procédé informatisé pour identifier une ou plusieurs paire(s) de gènes, sélectionnée(s) à partir d'un jeu de N gènes, laquelle paire ou lesquelles paires est/sont statistiquement associée(s) à un pronostic d'une pathologie potentiellement fatale,
le procédé utilisant des données de test qui, pour chaque sujet k d'un jeu de K* sujets qui souffrent de la pathologie, indiquent (i) un temps de survie du sujet k, et (ii) pour chaque gène i, une valeur d'expression de gène correspondante $y_{i,k}$ du sujet k ;
le procédé comprenant :

   (i) pour chaque paire d'une pluralité de paires des N gènes (i, j avec $i \neq j$) :

      (a) le partitionnement (2) des K* sujets selon deux sous-jeux en fonction de si $\log(y_{i,k}) - \log(y_{j,k})$ est respectivement au-delà ou en-deçà d'une valeur de coupure $c_{i,j}$ ;
      (b) l'ajustement par calcul informatique (3 ; 12) des temps de survie correspondants de l'un des sous-jeux des sujets sur le modèle de régression aléatoire proportionnelle de Cox, ledit ajustement générant un paramètre de régression respectif $\beta_{i,j}$ ; et
      (c) l'obtention, à partir du paramètre de régression $\beta_{i,j}$, d'une valeur de pertinence qui est indicative d'une pertinence de pronostic de la paire de gènes i,j ; et

   (ii) l'identification (4 ; 13) d'une ou de plusieurs des paires de gènes i,j pour laquelle ou lesquelles les valeurs de pertinence correspondantes présentent la pertinence de pronostic la plus élevée.

2. Procédé informatisé pour découvrir une ou plusieurs paire(s) de gènes, sélectionnée(s) à partir d'un jeu de N gènes, laquelle paire ou lesquelles paires est/sont statistiquement associée(s) à un pronostic d'une pathologie potentiellement fatale, le procédé incluant :

   (i) pour chacun d'une pluralité de jeux de sujets, l'identification d'un jeu respectif de paires de gènes parmi le jeu de N gènes au moyen d'un procédé selon la revendication 1 ; et
   (ii) la découverte (13) d'une ou de plusieurs paire(s) de gènes qui est/sont commune(s) à la pluralité de jeux de paires de gènes.

3. Procédé informatisé selon la revendication 2, incluant en outre la vérification de la pertinence de pronostic des paires de gènes découvertes en utilisant des données qui décrivent les temps de survie et les niveaux d'expression de gène correspondants d'un autre jeu de sujets.

4. Procédé informatisé selon la revendication 2, incluant en outre l'application d'un algorithme d'amplification (15) pour sélectionner une ou plusieurs paire(s) de gènes parmi les paires de gènes qui sont communes à la pluralité de jeux de paires de gènes.

5. Procédé informatisé selon la revendication 1, dans lequel la pathologie est un cancer, et les niveaux d'expression proviennent d'échantillons de tumeurs respectives chez les K* sujets.

6. Procédé selon la revendication 5, dans lequel la pathologie est un cancer du sein.

7. Procédé selon la revendication 1, dans lequel le temps de survie est un temps de survie jusqu'au décès.

8. Procédé selon la revendication 1, dans lequel le temps de survie est un temps de survie sans métastases d'un cancer.

9. Procédé informatisé d'obtention d'une information concernant un premier sujet en relation avec une pathologie potentiellement fatale, le procédé incluant :

   (i) l'identification, au moyen d'un procédé selon la revendication 1, à partir d'un jeu de N gènes, d'une ou de plusieurs paire(s) de gènes qui est/sont statistiquement associée(s) à un pronostic de la pathologie ;
   (ii) pour chacune des une ou plusieurs paire(s) de gènes identifiée(s) i, j, l'obtention de valeurs d'expression de gène correspondantes $y_i$ et $y_j$ du premier sujet ; et
   (iii) l'obtention de ladite information en utilisant un rapport des valeurs d'expression de gène obtenues.

**10.** Procédé informatisé selon la revendication 9, dans lequel ladite information est un pronostic pour le premier sujet qui souffre de la pathologie, une vulnérabilité du premier sujet à la pathologie, une prédiction de la récurrence de la pathologie, ou un traitement recommandé pour la pathologie.

**11.** Système informatique agencé de manière à réaliser un procédé selon l'une quelconque des revendications 1 à 10.

**12.** Produit de programme informatique comprenant un logiciel qui peut être exécuté par un ordinateur de manière à forcer l'ordinateur à réaliser un procédé selon l'une quelconque des revendications 1 à 10.

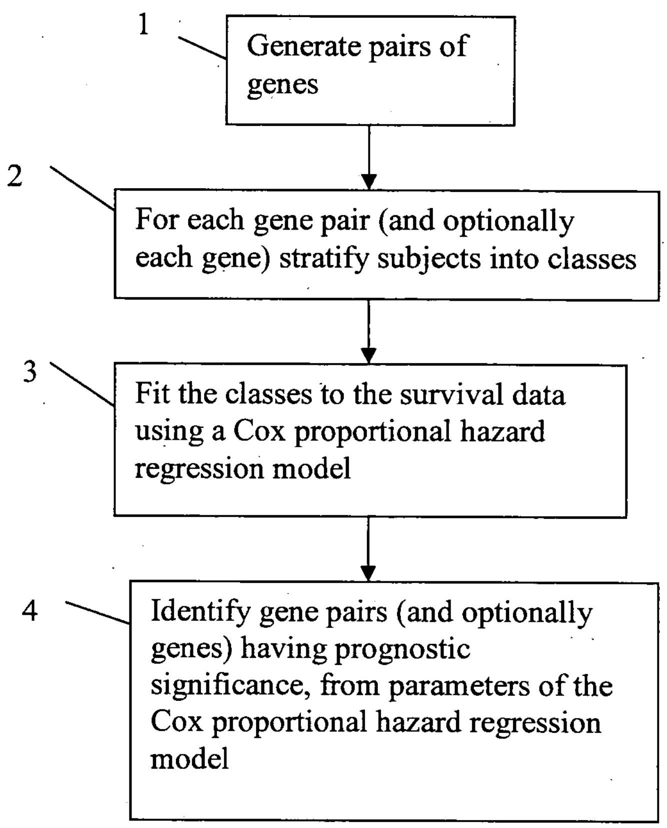
1
Generate pairs of genes
2
For each gene pair (and optionally each gene) stratify subjects into classes
3
Fit the classes to the survival data using a Cox proportional hazard regression model
4
Identify gene pairs (and optionally genes) having prognostic significance, from parameters of the Cox proportional hazard regression model

Fig. 1

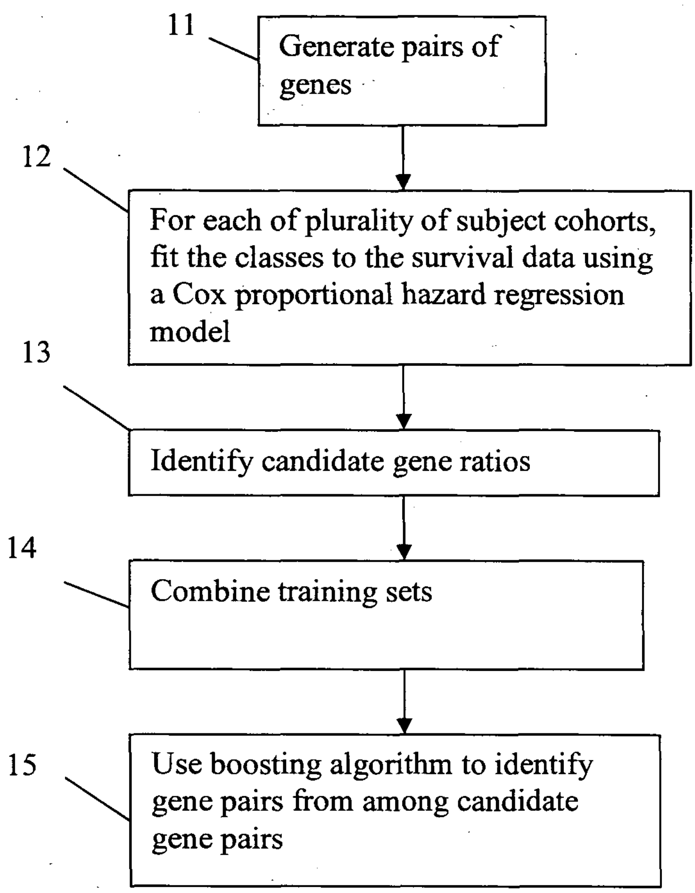
11
Generate pairs of genes
12
For each of plurality of subject cohorts, fit the classes to the survival data using a Cox proportional hazard regression model
13
Identify candidate gene ratios
14
Combine training sets
15
Use boosting algorithm to identify gene pairs from among candidate gene pairs

Fig. 2

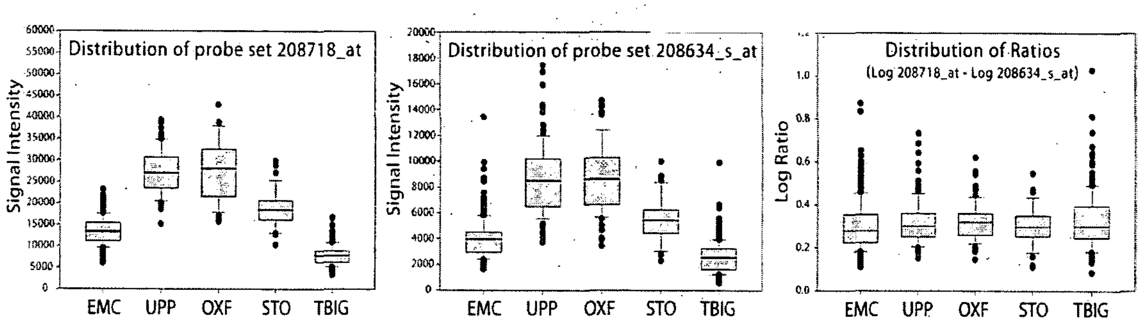


Fig. 3

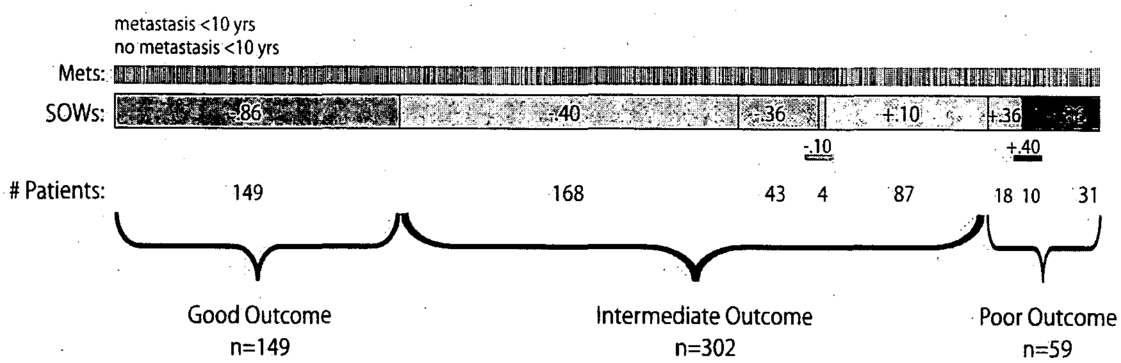


Fig. 4

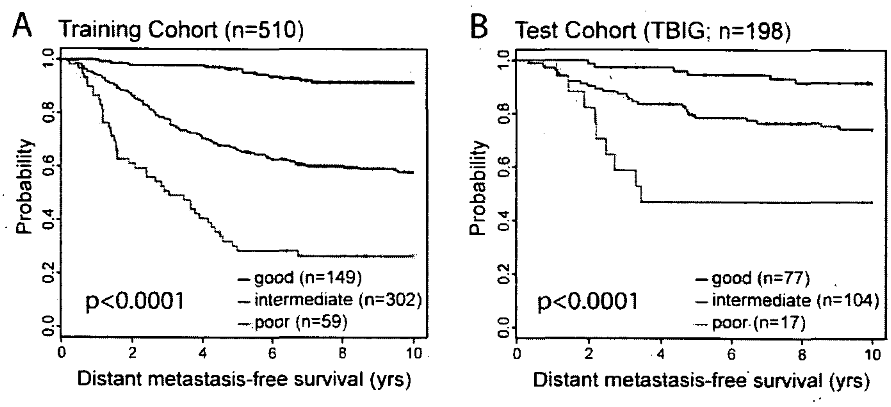


Fig. 5

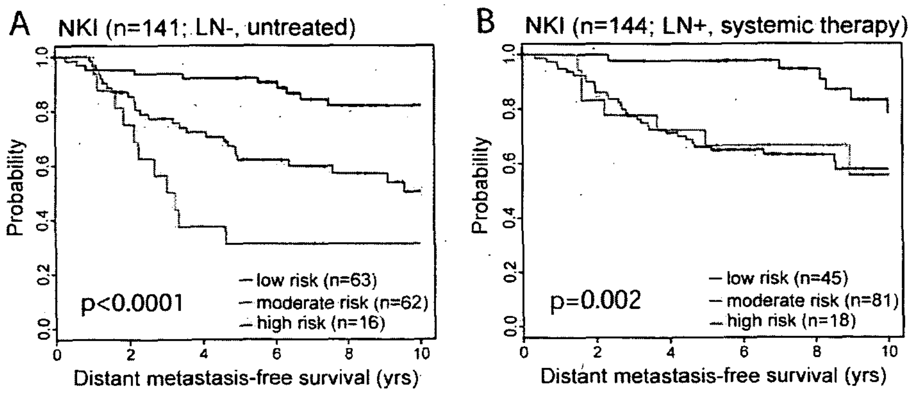
A NKI (n=141; LN-, untreated)
Probability
1.0
0.8
0.6
0.4
0.2
0.0
p<0.0001
low risk (n=63)
moderate risk (n=62)
high risk (n=16)
0
2
4
6
8
10
Distant metastasis-free survival (yrs)
B NKI (n=144; LN+, systemic therapy)
Probability
p=0.002
low risk (n=45)
moderate risk (n=81)
high risk (n=18)
Distant metastasis-free survival (yrs)

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61158948 B


### Non-patent literature cited in the description


- Identification of essential genes and gene pairs associated with survival time of cancer patients. **MOTAKIS et al.** BIOCOMP. CSREA Press, 2007, 753-759
- **V. KUZNETSOV et al.** Low and high-aggressive genetic breast cancer subtypes and significant survival gene signatures. *IJCNN,* 2008, 4151-4156
- **ROSELL et al.** BRCA1: a novel prognostic factor in resected non-small-cell lung cancer. *PLOS ONE,* vol. 2 (11), e1129-1
- **BROËT et al.** Identifying gene expression changes in breast cancer that distinguish early and late relapse among uncured patients. *Bioinformatics,* vol. 22 (12), 1477-1485
- **GOETZ et al.** A Two-Gene Expression Ratio of Homeobox 13 and Interleukin-17B Receptor for Prediction of Recurrence and Survival in Women Receiving Adjuvant Tamoxifen. *Clinical Cancer Research,* 2006, vol. 12 (7), 2080-2087
- **SAMBROOK ; RUSSEL.** Molecular Cloning: A Laboratory Manual. Cold Springs Harbor Laboratory, 2001
- **BRESLOW, N.E.** Covariance analysis of censored survival data. *Biometrics,* 1974, vol. 30, 89-99
- **COX, D.R. ; SNELL, E.J.** A general definition of residuals (with discussion). *Journal of the Royal Statistical Society,* 1968, vol. 30, 248-265
- **COX R.D. ; OAKES, D.** Analysis of Survival Data. Chapman and Hall, 1984
- **DESMEDT C ; PIETTE F ; LOI S ; WANG Y ; LALLEMAND F ; HAIBE-KAINS B ; VIALE G ; DELORENZI M ; ZHANG Y ; D'ASSIGNIES MS.** TRANSBIG Consortium. Strong time dependence of the 76-gene prognostic signature for node-negative breast cancer patients in the TRANSBIG multicenter independent validation series. *Clin Cancer Res,* 01 June 2007, vol. 13 (11), 3207-14
- **EFRON, B. ; TIBSHIRANI, R.J.** An Introduction to the Bootstrap. Chapman and Hall, 1994
- **IVSHINA, A.V. ; GEORGE, J. ; SENKO, O et al.** Genetic reclassification of histologic grade delineates new clinical subtypes of breast cancer. *Cancer Research,* 2006, vol. 66, 10292-10301
- **KAPLAN, E. L. ; MEIER, P.** Nonparametric estimation from incomplete observations. *JASA,* 1958, vol. 53, 457-48
- **KUZNETSOV, V.A. ; SENKO, O.V. ; MILLER, L.D. ; IVSHINA, A.** Statistically Weighted Voting Analysis of Microarrays for Molecular Pattern Selection and Discovery Cancer Genotypes. *International Journal of Computer Science and Network Security,* 2006, vol. 6, 73-83
- **LOI S ; HAIBE-KAINS B ; DESMEDT C ; LALLEMAND F ; TUTT AM ; GILLET C ; ELLIS P ; HARRIS A ; BERGH J ; FOEKENS JA.** Definition of clinically distinct molecular subtypes in estrogen receptor-positive breast carcinomas through genomic grade. *J Clin Oncol.,* 01 April 2007, vol. 25 (10), 1239-46
- **LOUGHIN, T.M.** A residual bootstrap for regression parameters in proportional hazards model. *J. of Statistical and Computational Simulations,* 1995, vol. 52, 367-384
- **MOTAKIS, E. ; NASON, G.P. ; FRYZLEWICZ, P. ; RUTTER, G.A.** Variance stabilization and normalization for one-color microarray data using a data-driven multiscale approach. *Bioinformatics,* 2006, vol. 22, 2547-2553
- **MILLENAAR, F. F. ; OKYERE, J. ; MAY, S.T. ; VAN ZANTEN, M. ; VOESENEK, L.A.C.J. ; PETERS, A.J.M.** How to decide? Different methods of calculating gene expression from short oligonucleotide array data will give different results. *BMC Bioinformatics,* 2006, vol. 7 (137
- **PAWITAN, Y. ; BJOHLE, J. ; AMLER, L.** Gene expression profiling spares early breast cancer patients from adjuvant therapy: derived and validated in two population-based cohorts. *Breast Cancer Research,* 2005, vol. 7, R953-R964
- **WANG Y ; KLIJN JG ; ZHANG Y ; SIEUWERTS AM ; LOOK MP ; YANG F ; TALANTOV D ; TIMMERMANS M ; MEIJER-VAN GELDER ME ; YU J.** Gene-expression profiles to predict distant metastasis of lymphnode-negative primary breast cancer. *Lancet,* 19 February 2005, vol. 365 (9460), 671-9